# EUROPEAN PATENT SPECIFICATION

(11) **EP 3 944 873 B1**
(45) Date of publication and mention of the grant of the patent: **28.09.2022**
(21) Application number: 21196951.4
(22) Date of filing: 12.12.2018
(51) Int. Cl.: A61N 1/378, H02J 7/00, A61N 1/36, H02J 50/00, A61N 1/362, A61N 1/05

(54) **FIXING DEVICE OF WIRELESS CHARGER AND WIRELESS CHARGING DEVICE**
FIXIERVORRICHTUNG FÜR EIN DRAHTLOSES LADEGERÄT UND DRAHTLOSES LADEGERÄT
DISPOSITIF DE FIXATION DE CHARGEUR SANS FIL ET DISPOSITIF DE CHARGE SANS FIL

(30) Priority: 14.12.2017 CN 201711341212; 04.12.2018 CN 201811474609
(43) Date of publication of application: 02.02.2022
(62) Divisional of application: 18888136.1
(73) Proprietor: Sceneray Co., Ltd, Jiangsu 215123 (CN)
(72) Inventor: NING, Yihua, Suzhou, Jiangsu 215123 (CN); ZHANG, Fan, Suzhou, Jiangsu 215123 (CN); GAO, Minfang, Suzhou, Jiangsu 215123 (CN)
(74) Representative: Lavoix

(56) References cited:
- WO-A1-2017/165410
- US-A- 3 942 535
- US-A1- 2009 270 951

## Description

### TECHNICAL FIELD

The present invention relates to a fixing device of a wireless charger, particularly to a fixing device of a wireless charger for charging a rechargeable implantable medical device, and a wearable wireless charging device.

### BACKGROUND

Implantable medical devices are diverse and applied in a wide scope, for example, cardiac pacemakers and spinal cord stimulators, and implantable neurostimulators. The implantable medical products available in the market are mostly powered by high-energy-density lithium primary batteries, and mostly have a shorter service life. In recent years, along with the development of wireless technologies and lithium rechargeable battery technologies, developing implantable rechargeable medical devices with a longer service life has become a mainstream future development tendency.

The implantable medical devices are implanted into a patient's body and isolated from an in vitro charging device by a tissue such as skin, and they need to be charged wirelessly and percutaneously. The charging manner is generally based on an electromagnetic coupling principle, and an electromagnetic field is used to penetrate the patient's skin to transfer energy to the implanted medical device. Upon charging, a charging coil needs to be accurately and stably align with the implanted stimulator. If there is not an assistant tool, the patient needs to be hold the coil close to the chest motionlessly for one hour or a longer period of time while charging, which is a painful thing and very difficult to complete by the patient. Furthermore, misalignment is likely to be caused without notice, which will cause a lower charging efficiency even failure to charge.

A fixing device of a portable wireless charger in the prior art is a shoulder strap form and includes a shoulder strap portion, a host sheath and a coil sheath. The shoulder strap is worn on the patient's body, and the charging coil and charger are placed in the coil sheath and host sheath respectively. Such a portable charging device has problems such as inconvenient wearing and detachment, and cannot be conveniently used by the patient.

US 3942535 represents the closest prior art to the subject-matter of independent claim 1 and discloses a fixing device of a wireless charger for an implanted medical device according to the preamble of said claim.

In view of the above problems, it is necessary to make further improvements with respect to drawbacks in the prior art.

### SUMMARY

An object of the present invention is to provide a fixing device of a wireless charger for charging a rechargeable implanted medical device. The fixing device of the wireless charger is simple to wear and quick and convenient to put on and put off.

To achieve the above object, the present invention provides a fixing device of a wireless charger for an implanted medical device according to claim 1.

As a further improvement of the embodiment of the present invention, wherein the front portion of the supporting member is provided with a charging opening, the charging opening corresponds to a position of the implanted medical device, and the charger fixing seat can be adjusted in the charging opening.

As a further improvement of the embodiment of the present invention, wherein the adjustment strap comprises a positioning strap connecting the shoulder portion of the supporting member or the rear portion of the supporting member and the charger fixing seat and a fixing strap connecting the charger fixing seat with the front portion of the supporting member, and the fixing strap can be adjusted and locked at the front portion of the supporting member.

As a further improvement of the embodiment of the present invention, wherein the positioning strap is configured as an elastic extensible belt or provided with a length adjuster, and the length adjuster moves in a lengthwise direction of the positioning strap to adjust a distance between a connection end of the positioning strap connected to the supporting member and the charger fixing seat.

As a further improvement of the embodiment of the present invention, wherein the fixing buckle comprises a female hook-and-loop fastener belt and a male hook-and-loop fastener belt engaging therewith and having a fluffy surface, the female hook-and loop fastener belt is disposed on one of the fixing strap and the front portion of the supporting member, the male hook-and-loop fastener belt is disposed on the other of the fixing strap and the front portion of the supporting member, and the fixing strap is adjusted and locked through the separable engagement of the female hook-and-loop fastener belt and the fluffy surface to adjust the position of the charger fixing seat.

As a further improvement of the embodiment of the present invention, wherein the positioning strap is connected to a middle portion of the rear portion of the supporting member via a fixing snap and is rotatable relative to the supporting member.

As a further improvement of the embodiment of the present invention, wherein the positioning strap is separably engaged to the shoulder supporting portion or the rear portion of the supporting member, and the engagement position can be adjusted.

As a further improvement of the embodiment of the present invention, wherein the front portion of the supporting member comprises a left front sheet and a right front sheet, and the left front sheet and right front sheet are both provided with a charging opening and separably connected.

As a further improvement of the embodiment of the present invention, wherein the left front sheet and right front sheet are partially overlapped, and connected in a hook-and-loop manner.

As a further improvement of the embodiment of the present invention, wherein an outer side of one of the left front sheet and right front sheet is provided with a first male hook-and-loop fastener belt having a fluffy surface, an inner side of the other of the left front sheet and right front sheet is provided with a female hook-and-loop fastener belt engaging with the fluffy surface, an outer side is provided with a second male hook-and-loop fastener belt having the fluffy surface, and the other end of the adjustment strap is separably connected to the second male hook-and-loop fastener belt.

The present invention also provides a wireless charging device, wherein the wireless charging device comprising a wireless charger and the fixing device of the wireless charger according to one of the embodiments above, the wireless charger is detachably mounted to the charger fixing seat, and the wireless charger can generate a charging field to charge the implanted medical device.

As compared with the prior art, the advantageous effects of the present invention are as follows: the fixing device of the wireless charger is configured as a wearable supporting member having a vest structure so that the charger can be held close to the patient's body and the patient can use it conveniently. Furthermore, the use of the fixing device of the wireless charger substantially reduces the processing steps in charging the rechargeable pulse-type generator.

### BRIEF DESCRIPTION OF THE DRAWINGS

Some specific embodiments of the present disclosure will be described later in detail in an exemplary and unrestrictive manner with reference to figures. The same reference numbers in the figures denote the same or like members or portions. Those skilled in the art should appreciate that these figures are not necessarily drawn to scale.
FIG. 1 is a perspective view of a fixing device of a wireless charger in a first preferred embodiment of the present invention;
FIG. 2 is a perspective view of the fixing device of FIG. 1 as viewed from another aspect;
FIG. 3 is a perspective view of a fixing device of a wireless charger in a second preferred embodiment of the present invention;
FIG. 4 is a perspective view of the fixing device of FIG. 3 as viewed from another aspect;
FIG. 5 is a perspective view of a fixing device of a wireless charger in a third preferred embodiment of the present invention;
FIG. 6 is a perspective view of the fixing device of FIG. 5 as viewed from another aspect;
FIG. 7 is a perspective view of a fixing device of a wireless charger in a fourth preferred embodiment of the present invention;
FIG. 8 is a perspective view of the fixing device of FIG. 7 as viewed from another aspect;
FIG. 9 is a front view when a fixing device of a wireless charger in a fifth preferred embodiment of the present invention is worn on a patient's body, wherein a shoulder supporting portion is located on the left side;
FIG. 10 is a rear view when the fixing device of FIG. 9 is worn on a patient's body;
FIG. 11 is a front view when a fixing device of a wireless charger in a sixth preferred embodiment of the present invention is worn on a patient's body;
FIG. 12 is a rear view when the fixing device of FIG. 11 is worn on a patient's body;
FIG. 13 is a front view when a fixing device of a wireless charger in a seventh preferred embodiment of the present invention is worn on a patient's body;
FIG. 14 is a rear view when the fixing device of FIG. 13 is worn on a patient's body;
FIG. 15 is a front view of a fixing device of a wireless charger in an eighth preferred embodiment of the present invention;
FIG. 16 is a rear view of the fixing device of FIG. 15;
FIG. 17 is a front view of the fixing device of FIG. 15 in another use state;
FIG. 18 is a rear view of the fixing device of FIG. 17;
FIG. 19 is a perspective view of a fixing device of a wireless charger in a ninth preferred embodiment of the present invention;
FIG. 20 is a rear view of the fixing device of FIG. 19;
FIG. 21 is a front view of the fixing device of FIG. 19;
FIG. 22 is a front view of the fixing device of FIG. 19 in another use state;
FIG. 23 is a perspective view of a fixing device of a wireless charger in a tenth preferred embodiment of the present invention;
FIG. 24 is a rear view of the fixing device of FIG. 23;
FIG. 25 is an exploded view of a charger fixing seat and a front sheet of the fixing device of FIG. 23;
FIG. 26 is an exploded view of the charger fixing seat of the fixing device of Fig. 23;
FIG. 27 is a schematic view of a closure edge structure of the fixing device of FIG. 23.

### DETAILED DESCRIPTION

The present invention will be described in detail below in conjunction with specific embodiments shown in the figures. However, these embodiments are not intended to limit the present invention. Structural, methodological or functional variations made by those having ordinary skill in the art according to these embodiments are all included in the protection scope of the present invention.

The fixing device of the wireless charger according to the present invention is used to charge the rechargeable implantable medical device. The rechargeable implantable medical device is adapted for an implantable deep brain stimulation system (DBS) or other similar stimulation systems. The rechargeable implantable medical device is implanted into a patient body upon use and used to apply a pulse electrical stimulation to the patient.

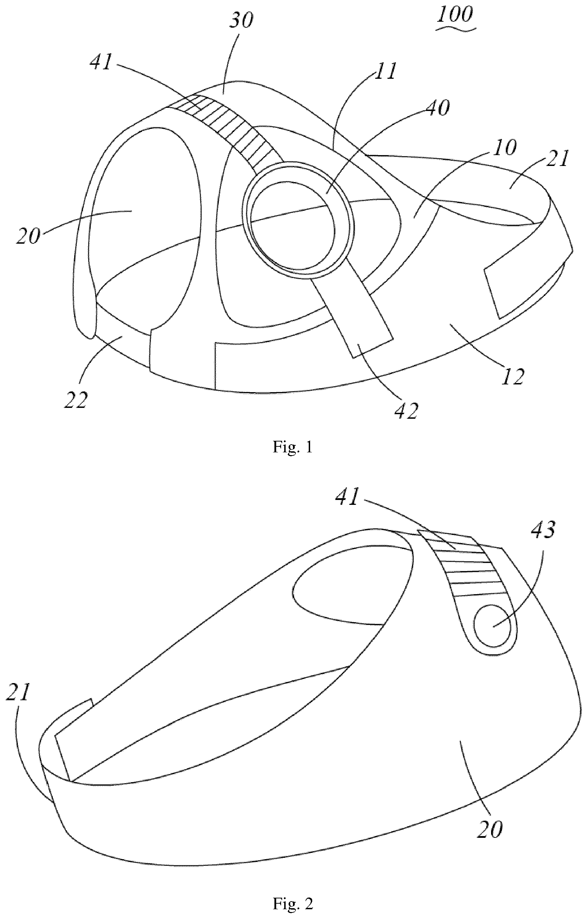

FIG. 1 and FIG. 2 show a first preferred embodiment of the fixing device of the wireless charger according to the present invention. The fixing device 100 of the wireless charger comprises a supporting member wearable on a patient's body and an adjustment structure connected to the supporting member, the supporting member employing a wearable supporting member such as a supporting member with a vest structure. A charger fixing seat 40 is connected to one of the supporting member and the adjustment structure. The adjustment structure comprises an adjustment strap and a fixing buckle engaging with the adjustment strap, one end of the adjustment strap is connected to the supporting member, the other end of the adjustment strap operably brings the supporting member or the charger fixing seat to be adjusted to a position where the charger fixing seat corresponds to the implantable medical device, and the position where the adjustment strap engages with the fixing buckle is fixed relative to the supporting member.

In the present embodiment, the charger fixing seat 40 is disposed on the adjustment strap, one end of the adjustment strap is connected to a shoulder portion or rear portion of the supporting member, and the other end of the adjustment strap operably brings the charger fixing seat to be adjusted to the position where the charger fixing seat corresponds to the implantable medical device, and is separably fastened to a front portion of the supporting member. The shoulder portion of the supporting member refers to a portion close to the patient's shoulder when the supporting member is worn on the patient's body. Likewise, the rear portion of the supporting member refers to a portion close to the patient's back when the supporting member is worn on the patient's body. The front portion of the supporting member refers to a portion close to the patient's chest when the supporting member is worn on the patient's body. Specifically, the supporting member comprises a shoulder supporting portion 30, and a front sheet 10 and a rear sheet 20 respectively connected to the front and rear of the shoulder supporting portion 30. The shoulder supporting portion 30 is a portion close to the patient's shoulder when the supporting member is worn on the patient's body and may be regarded as the shoulder portion of the supporting member; the front sheet 10 is a portion close to the patient's chest when the supporting member is worn on the patient's body and may be regarded as the front portion of the supporting member; the rear sheet 20 is a portion close to the patient's back when the supporting member is worn on the patient's body and may be regarded as the rear portion of the supporting member. Bottom sides of the front sheet 10 and the rear sheet 20 are connected via a left connecting portion 21 and a right connecting portion 22. The left connecting portion 21 and the right connecting portion 22 are used to form a shoulder cuff to be supported under the patient's armpit. One end of the adjustment strap is connected to the shoulder supporting portion 30 or the rear sheet 20 of the shoulder supporting member. The front sheet 10 is provided with a charging opening 11. The other end of the adjustment strap operably brings the charger fixing seat 40 to be adjusted to a position in the charging opening 11 corresponding to the implantable medical device, that is to say, the charging opening 11 corresponds to the position of the implantable medical device.

In the present embodiment, the fixing buckle comprises a female hook-and-loop fastener belt and a male hook-and-loop fastener belt engaging therewith and having a fluffy surface. The female hook-and-loop fastener belt is disposed on one of the adjustment strap and the front sheet 10, and the male hook-and-loop fastener belt is disposed on the other of the adjustment strap and the front sheet 10. The adjustment strap is fixed through the separable engagement of hook-and-loop and fluffy surface to adjust the position of the charger fixing seat 40. In addition, the shoulder supporting portion 30 and the front sheet 10 and rear sheet 20 are directly connected together, and preferably the shoulder supporting portion 30 and the front sheet 10 and rear sheet 20 are an integral structure. Certainly, in other implementable solutions, the shoulder supporting portion 30 may also be configured to be an extensible belt, i.e., the front sheet 10 and rear sheet 20 are indirectly connected via a telescopic belt. In addition, the front 10 and rear sheet 20 may also be disposed symmetrical relative to the shoulder supporting portion 30 to facilitate manufacture. The supporting member is entirely constructed as a single-shoulder vest structure, i.e., only one shoulder supporting portion 30 is provided, namely, the shoulder supporting portion 30 on the right side. The charging opening 11 may be disposed on one side with the shoulder supporting portion 30.

The left connecting portion 21 may also be configured as a belt integral with the rear sheet, and it may have a free end on which is provided a second female hook-and-loop fastener belt. The front sheet 10 is provided with a male hook-and-look fastener belt having a fluffy surface 12. The free end of the left connecting potion 21 may be adjusted according to the patient's body to a suitable position to engage with the fluffy surface 12 of the front sheet 10. The fluffy surface 12 is configured to be strip-shaped, triangular or arcuate and continuously distributed along the lower side of the front sheet. The fluffs of the fluffy surface are formed of fabric with fine fluffs protruding. The fine fluffs have tiny protrusions which are in the shape of short fluffs or pointed fluffs or fingers or branches or loops or honeycombs. The right connecting portion 22 may be configured to be an extensible connecting portion made of an extensible material or extensible structure, and may also be a belt shape. Both ends of the right connecting portion 22 are respectively connected to the front sheet 10 and rear sheet 20, and the right connecting portion 22 may be adjusted through its own extensibility to adapt to different human bodies. The left connecting portion 21 and right connecting portion 22 may be configured to be the same or different structures.

Furthermore, the adjustment strap comprises a positioning strap 41 connected to a shoulder supporting portion 30 or rear sheet 20 and a fixing strap 42. In the present embodiment, the positioning strap 41 is connected to the shoulder supporting portion 30 or a position of the rear sheet 20 adjacent to the shoulder supporting portion 30. The fixing strap 42 is configured as the other end of the adjustment strap. The fixing strap 42 is separably connected to the front sheet 10. A preferable female hook-and-loop fastener belt may be disposed on the fixing strap 42 and engage with the fluffy surface 12 on the front sheet 10. The positioning strap 41 and fixing strap 42 are respectively connected at opposite sides of the fixing seat 40. Furthermore, the positioning strap 41 and fixing strap 42 may be arranged to detachably connect with the fixing seat. The shape of the fixing seat matches that of the wireless charger to facilitate the wireless charger to be mounted and held to the fixing seat. The end of the positioning strap 41 connected to the shoulder supporting portion 30 or rear sheet 20 is connected via a fixing snap 43 disposed on the shoulder supporting portion 30 or rear sheet 20 so that the positioning strap 41 can be adjusted relative to the position of the supporting member.

In addition, to mount the wireless charger on the charger fixing seat more reliably, an elastic band for retaining the wireless charger may be disposed on the charger fixing seat. For example, the elastic band may be hooked to both sides of the charger fixing seat to limit the displacement of the wireless charger.

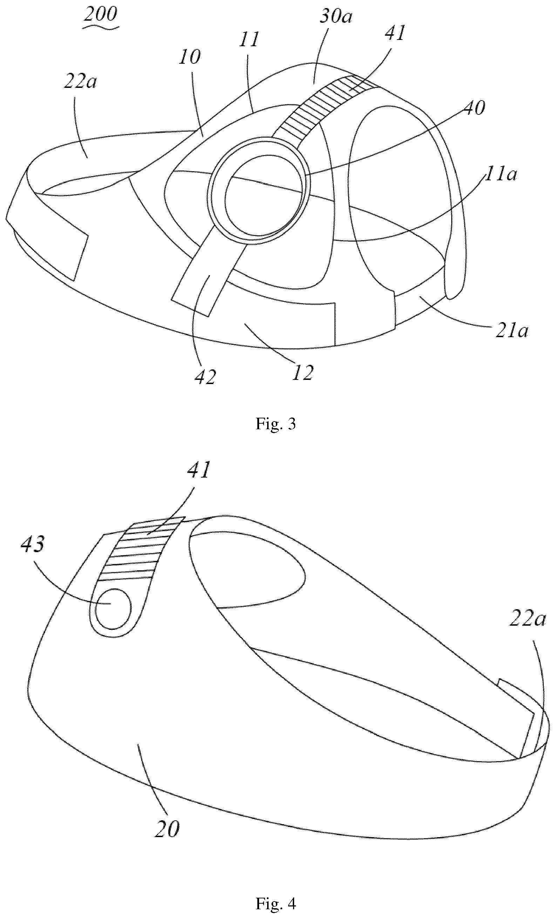

FIG. 3 and FIG. 4 show a second preferred embodiment of the fixing device of the wireless charger of the present invention. Components of the fixing device 200 of the wireless charger which are denoted with the same reference numbers as the first embodiment have the same structures and functions and will not be described in detail any more. Different from the first embodiment, one shoulder supporting portion is disposed as the shoulder supporting portion 30a on the left side. A left connecting portion 21a in the present embodiment is structurally similar to the right connecting portion 22 in the first embodiment, and a right connecting portion 22a is structurally similar to the left connecting portion 21 in the first embodiment. A charging opening 11a is disposed on the left side of the supporting member. That is to say, the fixing device 100 in the first embodiment and the fixing device 200 in the second embodiment may be regarded as symmetrical structures.

According to the above two embodiments, the supporting member structured as a single vest may be disposed as left-side single-shoulder or right-side single-shoulder. The left connecting portion and right connecting portion are structured the same and interchangeable. The female hook-and-loop fastener belt and the male hook-and-loop fastener belt having the fluffy surface 12 may also be disposed interchangeable, i.e., the front sheet 10 is provided with the female hook-and-loop fastener belt, and free ends of the fixing strap 42, the left connecting portion 21 and right connecting potion 22a are provided with the male hook-and-loop fastener belt engaging with the female hook-and-loop fastener belt and having the fluffy surface, which all can be implemented. Certainly, the interchange of the left-side single-shoulder or right-side single-shoulder may also be implemented through one of single-shoulder structural forms, e.g., the supporting member may be arranged to be wearable from inside or outside so that the left-shoulder vest may be worn reversely as the right-shoulder vest. As such, the male hook-and-loop fastener belt having the fluffy surface 12 may be disposed on inner and outer sides of the front sheet, and preferably disposed at the lower side of the front sheet. The inner and outer sides of the left connecting portion 21 or right connecting portion 22a are both provided with the female hook-and-loop fastener belt engaging with the fluffy surface 12. Furthermore, the inner and outer sides of the fixing strap 42 may also be provided with the female hook-and-loop fastener belt to engage with the fluffy surface 12. In addition, the fixing snap 43 may also be disposed on both inner and outer sides of the supporting member so that the positioning strap 41 can be conveniently connected when the supporting member is worn reversely.

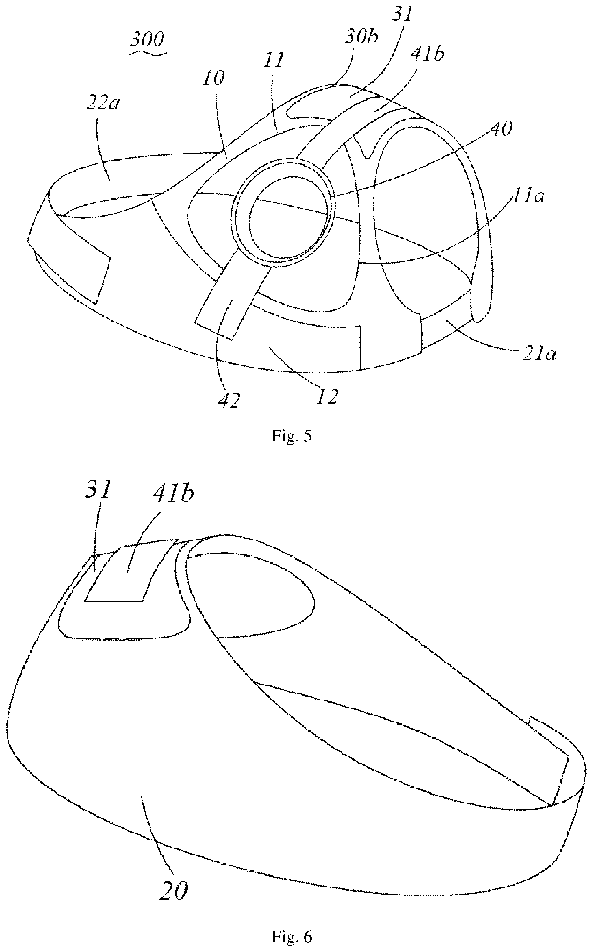

FIG. 5 and FIG. 6 show a third preferred embodiment of the fixing device of the wireless charger of the present invention. Components of the fixing device 300 of the wireless charger which are denoted with the same reference numbers as the second embodiment have the same structures and functions and will not be described in detail any more. Different from the second embodiment, the shoulder supporting portion 30b is also provided with the shoulder fluffy surface 31. Correspondingly, the positioning strap 41b is provided with the female hook-and-loop fastener belt. After the position of the charger fixing seat 40 is adjusted through the fixing strap 42, the positioning strap 41b is fixed relative to the position of the shoulder supporting portion 41b in a way that the female hook-and-loop fastener belt on the positioning strap 41b engages with the shoulder fluffy surface 31 on the shoulder supporting portion 30b, thereby preventing the displacement of the charger fixing seat 40 during use. Alternatively, the positioning strap 41b is fixed first, and then the fixing strap 42 is connected to the fluffy surface 12 of the front sheet 10.

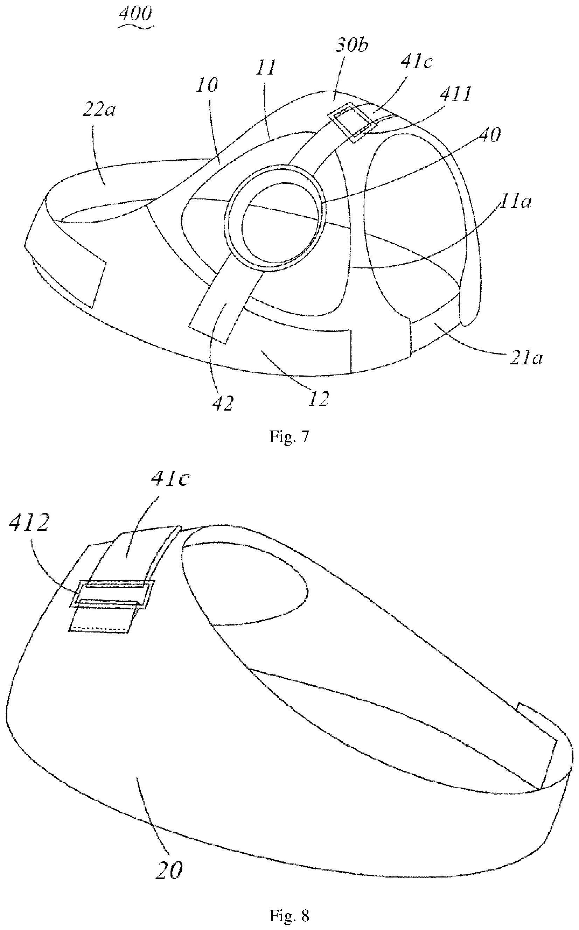

FIG. 7 and FIG. 8 show a fourth preferred embodiment of the fixing device of the wireless charger of the present invention. Components of the fixing device 400 of the wireless charger which are denoted with the same reference numbers as the second embodiment have the same structures and functions and will not be described in detail any more. Different from the second embodiment, the positioning strap 41c is configured adjustable in length. The positioning strap 41c is connected to a position of the rear sheet 20 adjacent to the shoulder fixing portion 30b. A fixing ring 412 is fixed on the shoulder fixing portion 30b or rear sheet 20. One end of the positioning strap 41c is connected to the fixing ring 412. An adjustable ring 411 is disposed on the positioning strap 41c. The length of the positioning strap 41c connected to the charger fixing seat 40 can be adjusted by operating the adjustable ring 411 and the positioning strap 41c. Hence, after the position of the charger fixing seat 40 is adjusted, the fixing strap 42 is connected to the fluffy surface 12 of the front sheet 10.

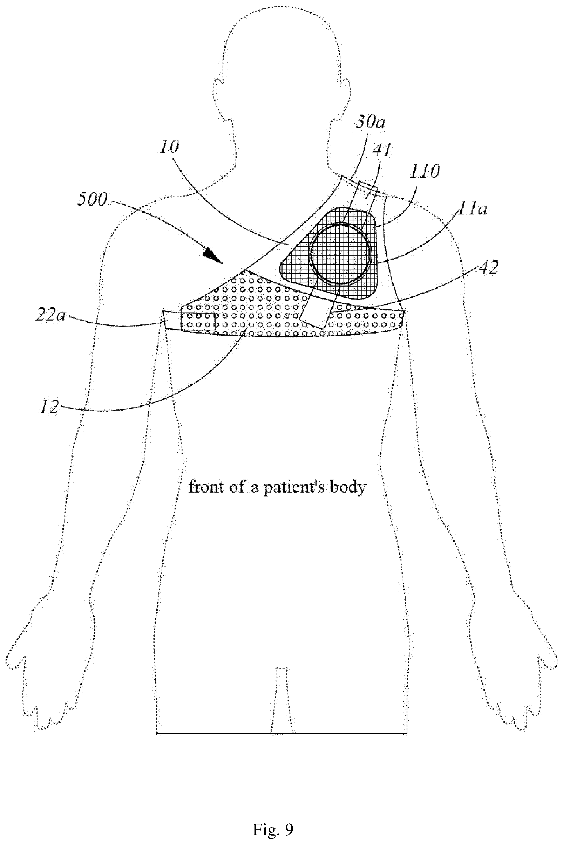

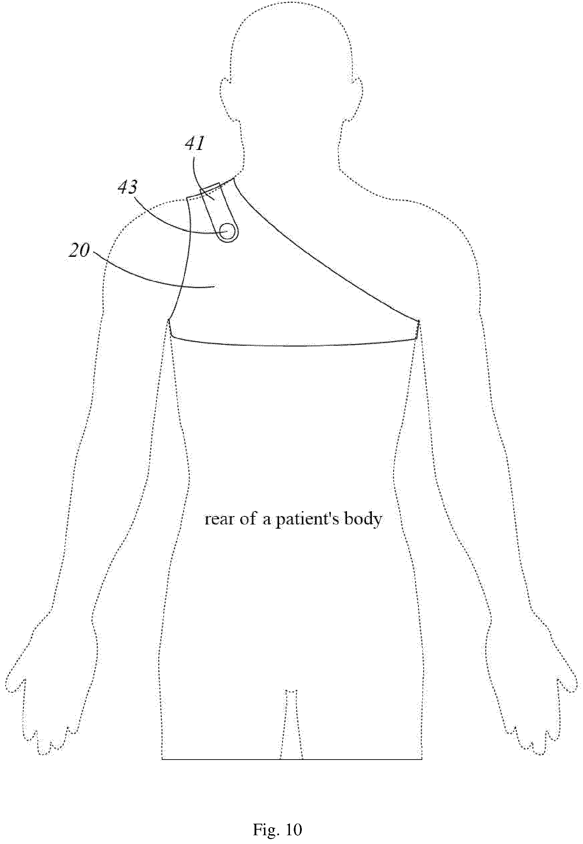

FIG. 9 and FIG. 10 show a fifth preferred embodiment of the fixing device of the wireless charger of the present invention. Components of the fixing device 500 of the wireless charger which are denoted with the same reference numbers as the second embodiment have the same structures and functions and will not be described in detail any more. Different from the second embodiment, the charging opening 11a is filled with a mesh-like fabric to facilitate heat dissipation upon charging. The charging opening 11a may be disposed on the left side or right side relative to the patient's body.

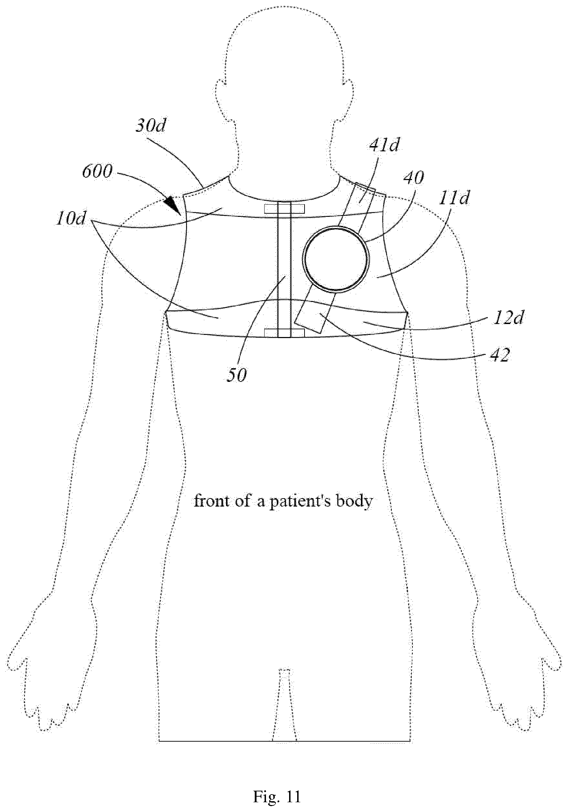

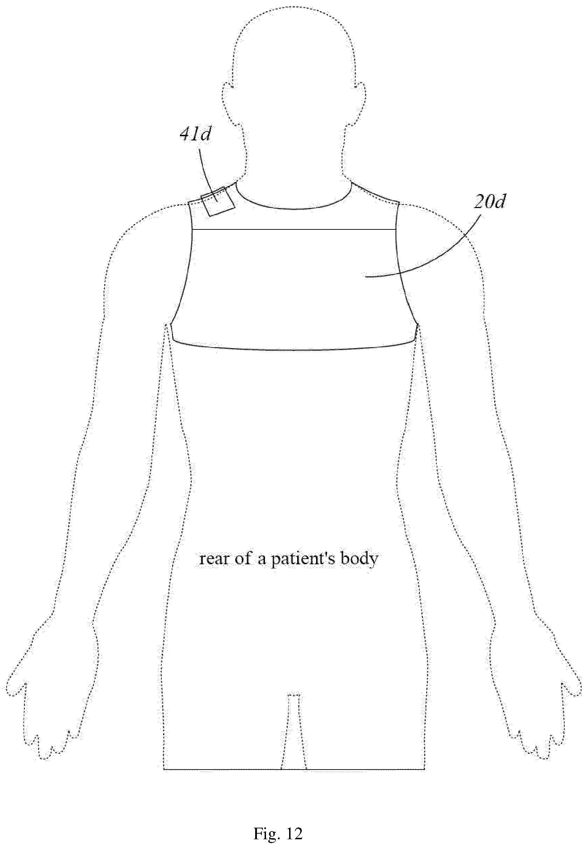

FIG. 11 and FIG. 12 show a sixth preferred embodiment of the fixing device of the wireless charger according to the present invention. The fixing device 600 of the wireless charger comprises a supporting member wearable on a patient's body and having a vest structure. The supporting member comprises a shoulder supporting portion 30d, and a front sheet 10d and a rear sheet 20d respectively connected to the front and rear of the shoulder supporting portion 30d. Bottom sides of the front sheet 10d and the rear sheet 20d are connected via a left connecting portion and a right connecting portion. The left connecting portion and the right connecting portion are used to form a shoulder cuff to be supported under the patient's armpit. The fixing device of the wireless charger further comprises an adjustment strap provided with a charger fixing seat 40. One end of the adjustment strap is connected to the shoulder supporting portion 30d or the rear sheet 20d of the shoulder supporting member. The front sheet 10d is provided with a charging opening 11d. The other end of the adjustment strap operably brings the charger fixing seat 40 to be adjusted to a position in the charging opening 11d corresponding to the implantable medical device, and is separably connected to the front sheet 10 of the shoulder supporting member.

In the present embodiment, the supporting member is entirely configured as a dual-shoulder vest structure, i.e., two shoulder supporting portions 30d are provided with one on each of the left side and right side. The charging opening 11d may also be arranged along both the left and right sides, e.g., the supporting member is entirely configured a left-right symmetrical structure. The shoulder supporting portion 30d and the front sheet 10d and rear sheet 20d are directly connected together, and preferably the shoulder supporting portion 30d and the front sheet 10d and rear sheet 20d are an integral structure. Furthermore, the left and right connecting portions may also be directly connected to the front sheet 10d and rear sheet 20d. The front sheet 10d is configured as a two-half structure from both sides. A slide fastener structure 50 is disposed between the both sides. The put-on and put-off of the supporting member can be achieved by operating the slide fastener structure 50. The front sheet 10d comprises an upper portion and a lower portion spaced apart, i.e., the charging opening 11d spaces the upper portion of the front sheet 10d from the lower portion of the front sheet 10d. The fluffy surface is disposed on the lower portion of the front sheet 10d. The positioning strap 41d may also be configured as an elastic band whose one end is fixed to the shoulder supporting portion 30d or rear sheet 20d. The configuration of the fixing strap 42 and the connection manner with the front sheet 10d may be the same as the previous embodiments and will not be detailed any more here.

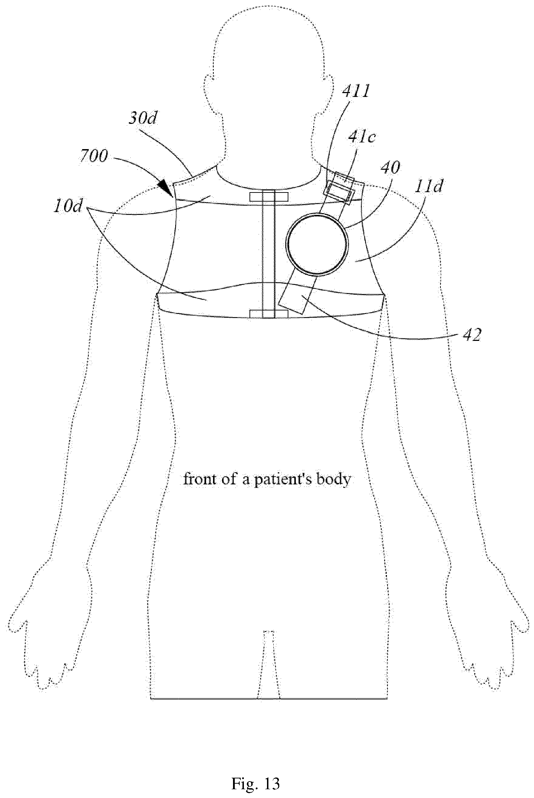

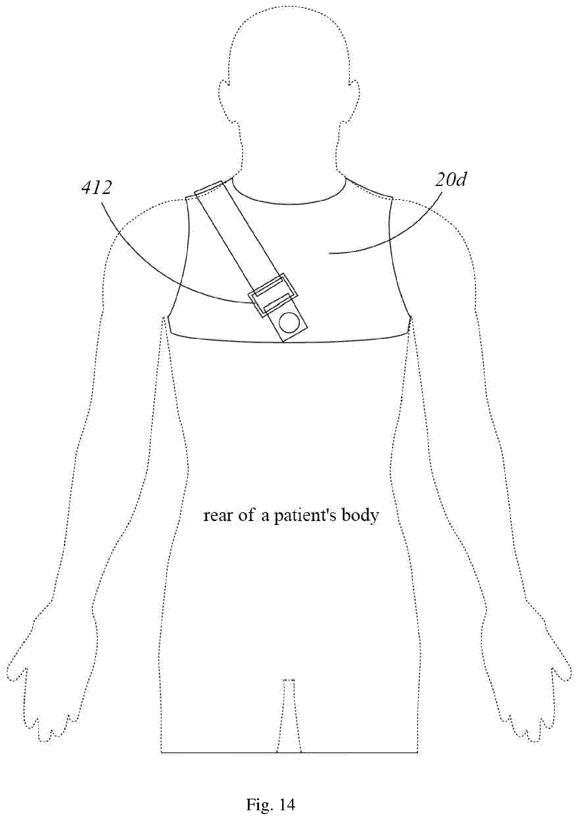

FIG. 13 and FIG. 14 show a seventh preferred embodiment of the fixing device of the wireless charger according to the present invention. Components of the fixing device 700 of the wireless charger which are denoted with the same reference numbers as the sixth embodiment have the same structures and functions and will not be described in detail any more. Different from the sixth embodiment, the positioning strap 41c is configured the same as the fourth embodiment and is adjustable in length. The shoulder fixing portion 30d or rear sheet 20d is fixed with a fixing ring 412. One end of the positioning strap 41c is connected to the fixing ring 412. The positioning strap 41c is provided with an adjusting ring 411. The length of the positioning strap 41c connected to the charger fixing seat 40 can be adjusted by operating the adjustment ring 411 and the positioning strap 41c. The adjustment ring 411 may be considered as a length adjuster, and it moves in a lengthwise direction of the positioning strap 41c to adjust the distance between a connection end of the positioning strap 41c connected to the supporting member and the charger fixing seat 40. Hence, after the position of the charger fixing seat 40 is adjusted, the fixing strap 42 is connected to the front sheet 10. The fixing ring 412 may be connected to the lower side of the rear sheet 20d via the fixing snap.

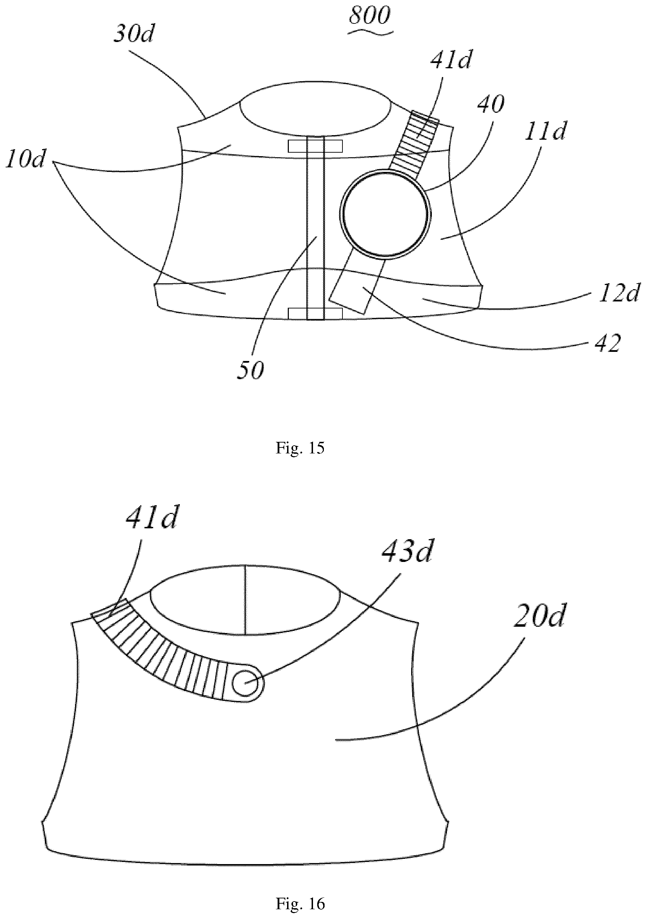

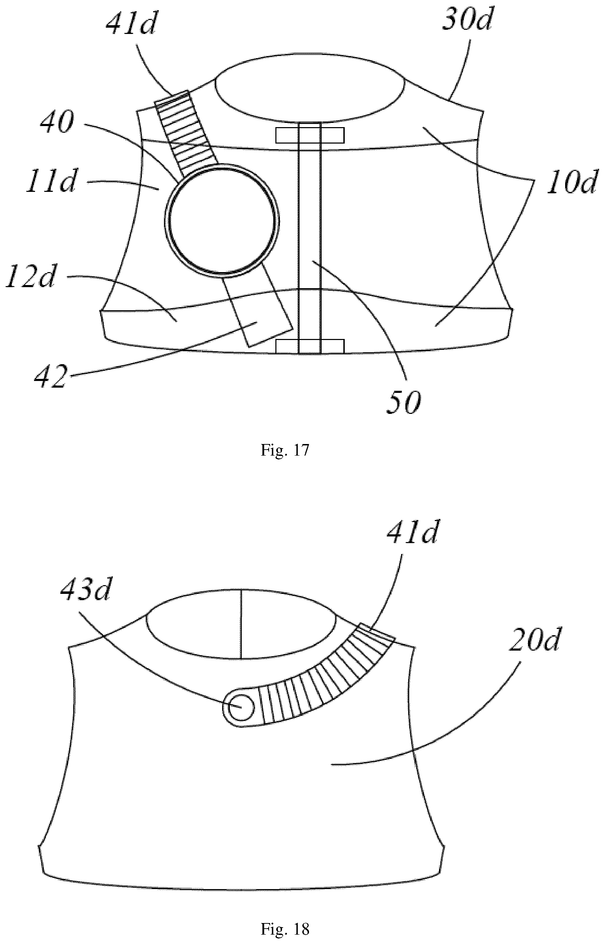

FIG. 15 through FIG. 18 show an eighth preferred embodiment of the fixing device of the wireless charger according to the present invention. Components of the fixing device 800 of the wireless charger which are denoted with the same reference numbers as the sixth embodiment have the same structures and functions and will not be described in detail any more. Different from the sixth embodiment, the positioning strap 41d is configured as a strap of an extensible material, and its one end connected to the rear sheet 20d is connected to the rear sheet 20d via a fixing snap 43d. The fixing snap 43d is disposed in the middle of the rear sheet 20d. Hence, the positioning strap 41d may selectively bypass from the left shoulder or right shoulder so that the charger fixing seat can be conveniently adjusted to the left side or right side of the patient's body.

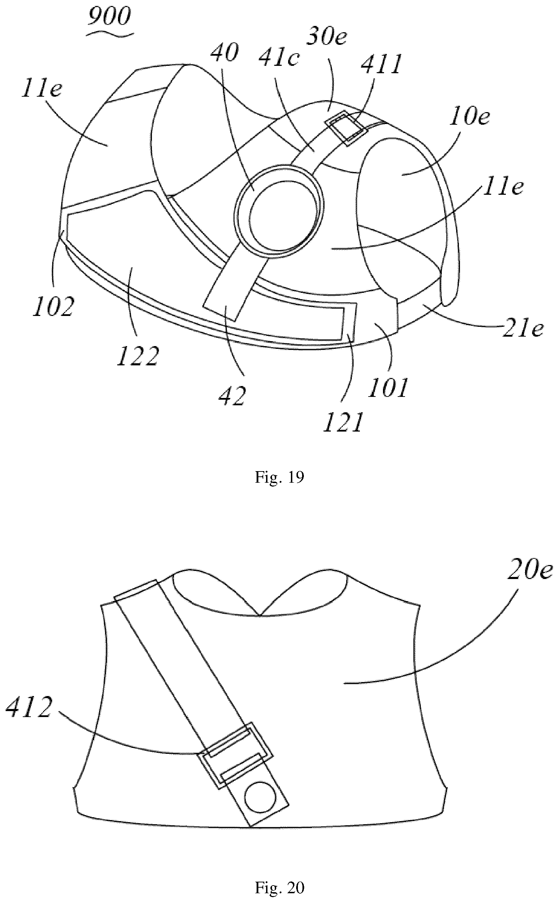

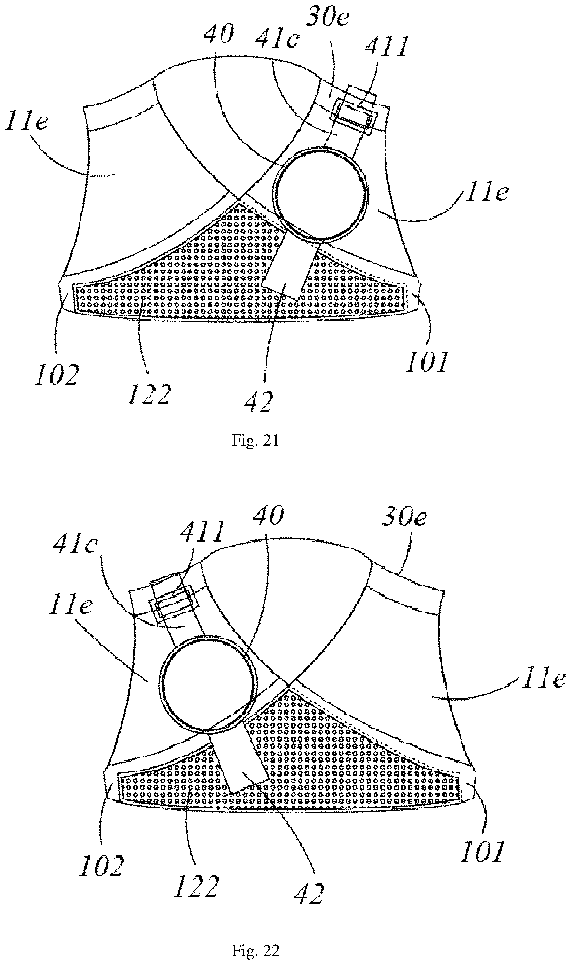

FIG. 19 through FIG. 22 show a ninth preferred embodiment of the fixing device of the wireless charger according to the present invention. The fixing 900 of the wireless charger comprises a supporting member wearable on a patient's body and having a vest structure. The supporting member comprises a shoulder supporting portion 30e, and a front sheet and a front sheet 20e respectively connected to the front and rear of the shoulder supporting portion 30e. Bottom sides of the front sheet and the rear sheet 20e are connected via a left connecting portion 21e and a right connecting portion. The left connecting portion 21e and the right connecting portion are used to form a shoulder cuff to be supported under the patient's armpit. The fixing device of the wireless charger further comprises an adjustment strap provided with a charger fixing seat 40. The configuration of the adjustment strap is similar to that of the sixth embodiment and will not be detailed any more here.

In the present embodiment, the supporting member is entirely configured as a dual-shoulder vest structure, i.e., two shoulder supporting portions 30e are provided with one on each of the left side and right side. The charging opening 11e may also be arranged along both the left and right sides, e.g., the supporting member is entirely configured a left-right symmetrical structure. The front sheet comprises a left front sheet 101 and a right front sheet 102 which are partially overlappable from left to right. The shoulder supporting portion 30d and the front sheet 10d and rear sheet 20d are directly connected together, and preferably the shoulder supporting portion 30e and the left front sheet 101 and right front sheet 102 and rear sheet 10e are an integral structure. The left connecting portion 21e and right connecting portion may be configured as an extensible belt connecting the left front sheet 101 with the rear sheet 10e and an extensible belt connecting the right front sheet 102 with the rear sheet 10e, respectively. The left front sheet 101 is provided with a first male hook-and-loop fastener belt having a fluffy surface 121. An outer side of the right front sheet 102 is provided with a second male hook-and-loop fastener belt having a fluffy surface 122. An inner side of the right front sheet 102 is provided with a third female hook-and-loop fastener belt engaging with the fluffy surface 121. The left front sheet 101 and right front sheet 102 achieve detachable connection of the two via the engagement of the third female hook-and-loop fastener belt and the fluffy surface 121 so that the size of the supporting member may be adjusted according to the patient's body. The fixing strap 42 may also be connected to the fluffy surface 122 of the right front sheet 102 via the female hook-and-loop fastener belt to achieve affixation of the charger fixing seat 40 relative to the supporting member. Certainly, the left-right overlappable manner of the left front sheet 101 and right front sheet 102 are interchangeable. Furthermore, the charger fixing seat 40 may be fixed in the charging opening on the left side or in the charging opening on the right side via the positioning strap 41c and fixing strap 42.

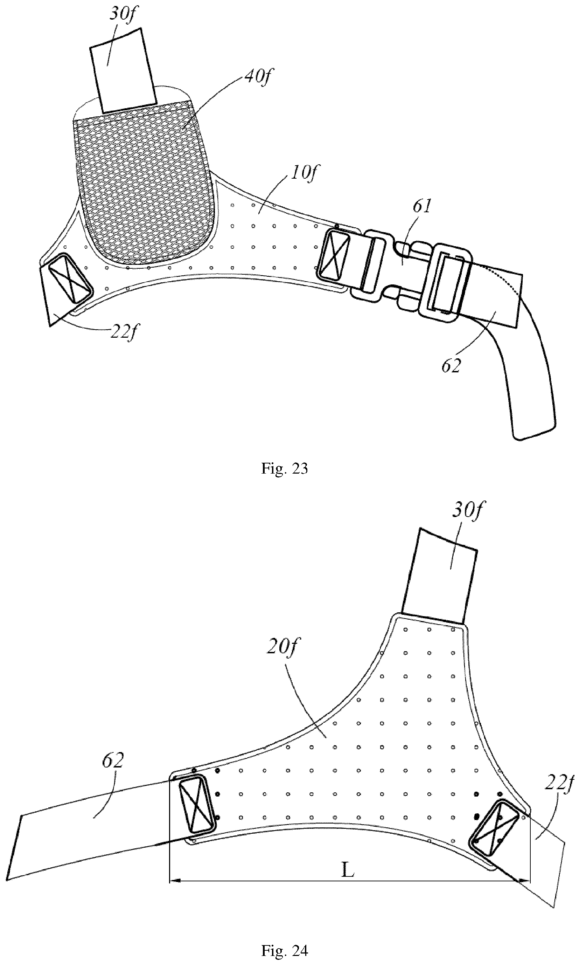

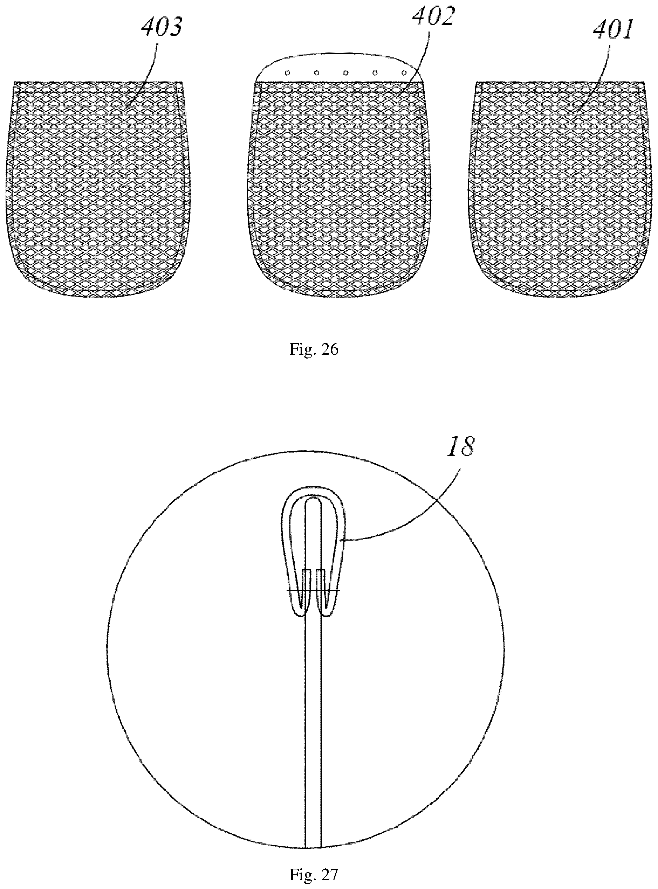

FIG. 23 through FIG. 27 show a tenth preferred embodiment of the fixing device of the wireless charger according to the present invention. The fixing device 1000 of the wireless charger comprises a supporting member wearable on a patient's body and an adjustment structure connected to the supporting member. A charger fixing seat 40f is connected to one of the supporting member and the adjustment structure. The adjustment structure comprises an adjustment strap 62 and a fixing buckle 61 engaging with the adjustment strap 62, one end of the adjustment strap 62 is connected to the supporting member, the other end of the adjustment strap 62 operably brings the supporting member or the charger fixing seat to be adjusted to a position where the charger fixing seat corresponds to the implantable medical device, and the position where the adjustment strap 62 engages with the fixing buckle 61 is fixed relative to the supporting member.

In the present embodiment, preferably, the charger fixing seat 40f is connected to the supporting member, one end of the fixing buckle 61 is connected to the supporting member, and the other end of the fixing buckle 61 is movably connected to the adjustment strap 62. The fixing buckle 61 comprises a male snap and a female snap which are detachably connected. The male snap and female snap are respectively connected to the supporting member and the adjustment strap 61. In other words, the patient may conveniently put on or put off the fixing device by opening the fixing buckle 61. The fixing buckle 61 may be configured as a plug-in buckle, a male buckle or a female buckle of the plug-in buckle is connected to the adjustment ring. The other end of the adjustment strap 62 passes through the adjustment ring and is configured as a free end. The position of the supporting member may be adjusted by pulling of the free end of the adjustment strap 62, and the fixing buckle 61 will bring the supporting member to move, until the charger fixing seat 40f corresponds to the position of the implantable medical device. Locking is achieved by releasing the adjustment strap 62, i.e., the engagement position of the adjustment strap 62 and the fixing buckle 61 is fixed relative to the supporting member.

Furthermore, the supporting member comprises a supporting portion 30f, and a front sheet 10f and a rear sheet 20f connected to the front and rear of the shoulder supporting portion 30f, respectively. Bottom sides of the front sheet 10f and the rear sheet 20f are connected via a left connecting portion and a right connecting portion. The shoulder supporting portion, the front sheet and rear sheet here are also regarded as the shoulder of the supporting member, the front of the supporting member and the rear of the supporting member. The left connecting portion and the right connecting portion are used to form a shoulder cuff to be supported under the patient's armpit. In the present embodiment, an example is taken in which the right connecting portion 22f forms the shoulder cuff, and the left connecting portion is configured as the adjustment structure. One end of the adjustment strap 62 is connected to the rear sheet 20f, and the fixing buckle 61 is connected between the front sheet 10f and the adjustment strap 62. As such, the position of the charger fixing seat 40f is adjusted via the left connecting portion serving as the adjustment structure, so that the structure of the whole fixing device is simpler, the costs are lower and the use is more convenient.

Preferably, the shoulder supporting portion 30f and the right connecting portion 22f are both configured as elastic extensible belts to facilitate adaptation to patients with different body shapes and facilitate the patients to adjust the position of the charger fixing seat 40f. Certainly, other structures with the length adjustable may also be employed. The charger fixing seat 40f is configured as a storage bag. The storage bag is connected between the shoulder supporting portion 30f and the front sheet 10f to facilitate receiving the wireless charger. The storage bag comprises an outer bag 401 located outside and an inner bag 403 located inside. A partition layer 402 is disposed between the outer bag 401 and inner bag 403. The shoulder supporting portion 30f is connected to the partition layer 402. As such, the storage bag is disposed both inside and outside the supporting member to achieve wearing of the fixing device from inside and from outside so that the charger fixing seat 40f is fixed to the left chest and right chest. Certainly, it is also possible that the storage bag comprises an outer bag located outside and an inner bag located inside. The inner bag and outer bag are communicated with each other, that is, the storage bag is communicated from outside and inside without a partition layer being disposed. More materials are saved with one storage bag which may receive the wireless charger from both inside and outside. The storage bag may be made of a mesh-like fabric.

To facilitate fixing the wireless charger in the storage bag, a female hook-and-loop fastener belt is disposed on one of an outer side of the storage bag and an inner side of the storage bag, a male hook-and-loop fastener belt having a fluffy surface is disposed on the other of the outer side of the storage bag and inner side of the storage bag, and the female hook-and-loop fastener belt separably engages with the male hook-and-loop fastener belt to close at least one portion of the bag mouth of the storage bag. It is also possible that one of the outer side of the storage bag and the shoulder supporting portion 30f is provided with a female hook-and-loop fastener belt, the other of the outer side of the storage bag and the shoulder supporting portion 30f is provided with a male hook-and-loop fastener belt having a fluffy surface, and the female hook-and-loop fastener belt separably engages with the male hook-and-loop fastener belt to close at least one portion of the bag mouth of the storage bag.

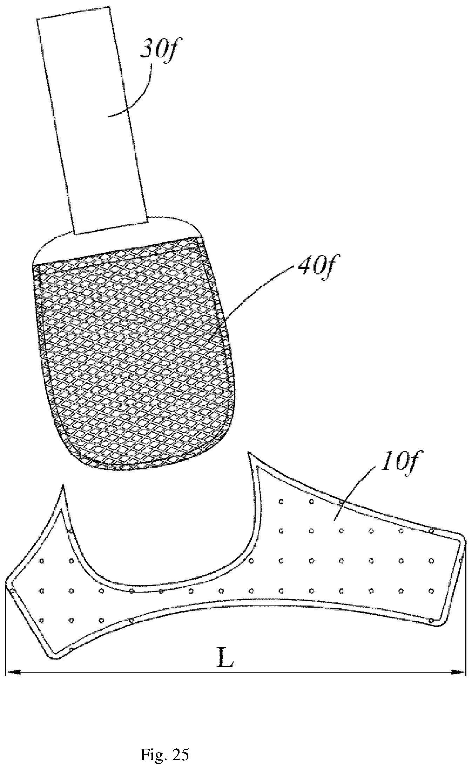

In addition, the front sheet 10f consists of an inner layer and an outer layer, and at least partial edge of the charger fixing seat is connected between the inner layer and outer layer so that the fixing device does not affect the patient's body when worn. In addition, the edge of each of the front sheet lOf, the rear sheet 20f and the charger fixing seat 40f may be provided with a closure edge structure 18 to make the patient more comfortable while wearing the fixing device and meanwhile make the appearance more aesthetic. In addition, as shown in FIG. 24 and FIG. 25, the overall length L of the front sheet and rear sheet is substantially in a range of 200-250mm, and the front sheet and rear sheet may also be made of a mesh-like fabric, no matter whether the fabric is elastic or not. The left connecting portion and right connecting portion disposed under the armpit has a length substantially in a range of 80-120mm, and a width in a range of 30-50mm. The shoulder supporting portion has a length substantially in a range of 90-130mm and a width in a range of 30-50mm. The adjustment strap has a length substantially in a range of 360-440mm and a width in a range of 30-50mm.

It should be understood that although the description is described according to the embodiments, not every embodiment only includes one independent technical solution, that such a description manner is only for the sake of clarity, that those skilled in the art should take the description as an integral part, and that the technical solutions in the embodiments may be suitably combined to form other embodiments understandable by those skilled in the art.

## Claims

1. A fixing device (100,200,300,400,500,600,700,800,900,1000) of a wireless charger for an implanted medical device, the fixing device comprising a supporting member wearable on a patient's body and an adjustment structure connected to the supporting member, a charger fixing seat (40,40f) is connected to one of the supporting member and the adjustment structure, the adjustment structure comprises an adjustment strap (41, 41b, 41c, 41d, 42, 62) and a fixing buckle (12,61) engaging with the adjustment strap (41, 41b, 41c, 41d, 42, 62), one end of the adjustment strap (41, 41b, 41c, 41d, 42, 62) is connected to the supporting member, the other end of the adjustment strap (41, 41b, 41c, 41d, 42, 62) operably brings the supporting member or the charger fixing seat (40,40f) to be adjusted to a position where the charger fixing seat (40,40f) corresponds to the implanted medical device, and a position where the adjustment strap engages with the fixing buckle can be adjusted and locked, wherein the charger fixing seat (40,40f) is disposed on the adjustment strap (41, 41b, 41c, 41d, 42), one end of the adjustment strap (41, 41b, 41c, 41d, 42) is connected to a shoulder portion or a rear portion of the supporting member, the other end of the adjustment strap (41, 41b, 41c, 41d, 42) operably brings the charger fixing seat (40,40f) to be adjusted to a position where the charger fixing seat (40,40f) corresponds to the implanted medical device, and the other end of the adjustment strap (41, 41b, 41c, 41d, 42) can be adjusted and locked at the front portion of the supporting member, **characterized in that**,
the supporting member comprises a left connecting portion (21, 21a, 21e) and a right connecting portion (22, 22a, 22f), bottom sides of a front portion of the supporting member and a rear portion of the supporting member are connected via the left connecting portion (21, 21a, 21e) and the right connecting portion (22, 22a, 22f), the left connecting portion (21, 21a, 21e) and the right connecting portion (22, 22a, 22f) are used to form a shoulder cuff to be supported under the patient's armpit, one end of the adjustment strap (41, 41b, 41c, 41d, 42) is connected to the shoulder portion of the supporting member or the rear portion of the supporting member, and the other end of the adjustment strap (41, 41b, 41c, 41d, 42) is adjusted and locked at the front portion of the supporting member.

2. The fixing device of a wireless charger according to claim 1, wherein the front portion of the supporting member is provided with a charging opening (11,11a,11d,11e), the charging opening (11,11a,11d,11e) corresponds to a position of the implanted medical device, and the charger fixing seat (40,40f) can be adjusted in the charging opening (11,11a,11d,11e).

3. The fixing device of a wireless charger according to claim 1, wherein the adjustment strap (41, 41b, 41c, 41d, 42) comprises a positioning strap (41, 41b, 41c, 41d) connecting the shoulder portion of the supporting member or the rear portion of the supporting member and the charger fixing seat (40,40f) and a fixing strap (42) connecting the charger fixing seat (40,40f) with the front portion of the supporting member, and the fixing strap (42) can be adjusted and locked at the front portion of the supporting member.

4. The fixing device of a wireless charger according to claim 3, wherein the positioning strap (41, 41b, 41c, 41d) is configured as an elastic extensible belt or provided with a length adjuster, and the length adjuster moves in a lengthwise direction of the positioning strap (41, 41b, 41c, 41d) to adjust a distance between a connection end of the positioning strap (41, 41b, 41c, 41d) connected to the supporting member and the charger fixing seat (40,40f).

5. The fixing device of a wireless charger according to claim 3, wherein the fixing buckle (12) comprises a female hook-and-loop fastener belt and a male hook-and-loop fastener belt engaging therewith and having a fluffy surface, the female hook-and loop fastener belt is disposed on one of the fixing strap (42) and the front portion of the supporting member, the male hook-and-loop fastener belt is disposed on the other of the fixing strap (42) and the front portion of the supporting member, and the fixing strap (42) is adjusted and locked through the separable engagement of the female hook-and-loop fastener belt and the fluffy surface to adjust the position of the charger fixing seat (40,40f).

6. The fixing device of a wireless charger according to claim 3, wherein the positioning strap (41, 41b, 41c, 41d) is connected to a middle portion of the rear portion of the supporting member via a fixing snap (42) and is rotatable relative to the supporting member.

7. The fixing device of a wireless charger according to claim 3, wherein the positioning strap (41, 41b, 41c, 41d) is separably engaged to the shoulder supporting portion or the rear portion of the supporting member, and the engagement position can be adjusted.

8. The fixing device of a wireless charger according to claim 2, wherein the front portion of the supporting member comprises a left front sheet (101) and a right front sheet (102), and the left front sheet (101) and right front sheet (102) are both provided with a charging opening (11,11a,11d,11e) and separably connected.

9. The fixing device of a wireless charger according to claim 8, wherein the left front sheet (101) and right front sheet (102) are overlappable left and right and connected in a hook-and-loop manner.

10. The fixing device of a wireless charger according to claim 8 , wherein an outer side of one of the left front sheet (101) and right front sheet (102) is provided with a first male hook-and-loop fastener belt having a fluffy surface (121), an inner side of the other of the left front sheet and right front sheet is provided with a female hook-and-loop fastener belt engaging with the fluffy surface (121), an outer side is provided with a second male hook-and-loop fastener belt having a fluffy surface (122), and the other end of the adjustment strap (42) is separably connected to the second male hook-and-loop fastener belt.

11. The fixing device of a wireless charger according to claim 1, wherein the supporting member comprises a shoulder supporting portion (30d), a front sheet (10d) and a rear sheet (20d) respectively connected to the front and rear of the shoulder supporting portion (30d), the supporting member is configured as a left-right symmetrical structure, the shoulder supporting portion (30d) and the front sheet (10d) and rear sheet (20d) are directly connected together, and the shoulder supporting portion (30d) and the front sheet (10d) and rear sheet (20d) are an integral structure.

12. The fixing device of a wireless charger according to claim 1, wherein the supporting member comprises a shoulder supporting portion (30d), a front sheet (10d) and a rear sheet (20d) respectively connected to the front and rear of the shoulder supporting portion (30d), the front sheet (10d) is configured as a two-half structure from both sides, a slide fastener structure (50)is disposed between the both sides, the put-on and put-off of the supporting member can be achieved by operating the slide fastener structure.

13. The fixing device of a wireless charger according to claim 5, wherein the supporting member comprises a shoulder supporting portion (30d), a front sheet (10d) and a rear sheet (20d) respectively connected to the front and rear of the shoulder supporting portion (30d), the front sheet (10d) comprises an upper portion and a lower portion spaced apart, the charging opening (11d) spaces the upper portion of the front sheet (10d) from the lower portion of the front sheet (10d), the fluffy surface is disposed on the lower portion of the front sheet (10d).

14. The fixing device of a wireless charger according to claim 11 or 12 or 13, wherein the positioning strap (41d) is configured as a strap of an extensible material, and one end of the positioning strap (41d) is connected to the rear sheet (20d) via a fixing snap (43d), the fixing snap (43d) is disposed in the middle of the rear sheet (20d), the positioning strap (41d) selectively bypass from the left shoulder or right shoulder so that the charger fixing seat can be adjusted to the left side or right side of the patient's body.

15. A wireless charging device, wherein the wireless charging device comprises a wireless charger and the fixing device (100,200,300,400,500,600,700,800,900,1000) of the wireless charger according to any of claims 1-14, the wireless charger is detachably mounted to the charger fixing seat (40,40f), and the wireless charger can generate a charging field to charge the implanted medical device.

## Patentansprüche

1. Befestigungsvorrichtung (100, 200, 300, 400, 500, 600, 700, 800, 900, 1000) eines drahtlosen Ladegeräts für eine implantierte medizinische Vorrichtung, die Befestigungsvorrichtung umfassend ein Halteelement, das am Körper eines Patienten getragen werden kann, und eine Einstellstruktur, die mit dem Halteelement verbunden ist, wobei ein Ladegerät-Befestigungssitz (40, 40f) mit einem von dem Halteelement oder der Einstellstruktur verbunden ist, wobei die Einstellstruktur einen Einstellriemen (41, 41b, 41c, 41d, 42, 62) und eine Befestigungsschnalle (12, 61), die in den Einstellriemen (41, 41b, 41c, 41d, 42, 62) eingreift, wobei ein Ende des Einstellriemens (41, 41b, 41c, 41d, 42, 62) mit dem Halteelement verbunden ist und das andere Ende des Einstellriemens (41, 41b, 41c, 41d, 42, 62) das Halteelement oder den einzustellenden Ladegerät-Befestigungssitz (40, 40f) in eine Position bringt, in der der Ladegerät-Befestigungssitz (40, 40f) der implantierten medizinischen Vorrichtung entspricht, und eine Position, in der der Einstellriemen mit der Befestigungsschnalle eingreift, eingestellt und arretiert werden kann, wobei der Ladegerät-Befestigungssitz (40, 40f) auf dem Einstellriemen (41, 41b, 41c, 41d, 42) angeordnet ist, ein Ende des Einstellriemens (41, 41b, 41c, 41d, 42) mit einem Schulterabschnitt oder einem hinteren Abschnitt des Halteelements verbunden ist, das andere Ende des Einstellriemens (41, 41b, 41c, 41d, 42) den einzustellenden Ladegerät-Befestigungssitz (40, 40f) funktionsfähig in eine Position bringt, in der der Ladegerät-Befestigungssitz (40, 40f) mit der implantierten medizinischen Vorrichtung übereinstimmt, und das andere Ende des Einstellriemens (41, 41b, 41c, 41d, 42) an dem vorderen Abschnitt des Halteelements eingestellt und arretiert werden kann, **dadurch gekennzeichnet, dass**
das Halteelement einen linken Verbindungsabschnitt (21, 21a, 21e) und einen rechten Verbindungsabschnitt (22, 22a, 22f) aufweist, Unterseiten eines vorderen Abschnitts des Halteelements und eines hinteren Abschnitts des Halteelements über den linken Verbindungsabschnitt (21, 21a, 21e) und den rechten Verbindungsabschnitt (22, 22a, 22f) verbunden sind, der linke Verbindungsabschnitt (21, 21a, 21e) und der rechte Verbindungsabschnitt (22, 22a, 22f) verwendet werden, um eine Schultermanschette zu bilden, die unter der Achselhöhle des Patienten gehalten werden soll, ein Ende des Einstellriemens (41, 41b, 41c, 41d, 42) mit dem Schulterabschnitt des Halteelements oder dem hinteren Abschnitt des Halteelements verbunden ist, und das andere Ende des Einstellriemens (41, 41b, 41c, 41d, 42) an dem vorderen Abschnitt des Halteelements eingestellt und arretiert wird.

2. Befestigungsvorrichtung eines drahtlosen Ladegeräts nach Anspruch 1, wobei der vordere Abschnitt des Halteelements mit einer Ladeöffnung (11, 11a, 11d, 11e) versehen ist, die Ladeöffnung (11, 11a, 11d, 11e) einer Position der implantierten medizinischen Vorrichtung entspricht und der Ladegerät-Befestigungssitz (40, 40f) in der Ladeöffnung (11, 11a, 11d, 11e) eingestellt werden kann.

3. Befestigungsvorrichtung eines drahtlosen Ladegeräts nach Anspruch 1, wobei der Einstellriemen (41, 41b, 41c, 41d, 42) einen Positionierungsriemen (41, 41b, 41c, 41d), der den Schulterabschnitt des Halteelements oder den hinteren Abschnitt des Halteelements und den Ladegerät-Befestigungssitz (40, 40f) verbindet, und einen Befestigungsriemen (42), der den Ladegerät-Befestigungssitz (40, 40f) mit dem vorderen Abschnitt des Halteelements verbindet, umfasst und der Befestigungsriemen (42) an dem vorderen Abschnitt des Halteelements eingestellt und arretiert werden kann.

4. Befestigungsvorrichtung eines drahtlosen Ladegeräts nach Anspruch 3, wobei der Positionierungsriemen (41, 41b, 41c, 41d) als ein elastischer dehnbarer Gurt konfiguriert oder mit einem Längeneinsteller versehen ist, und der Längeneinsteller sich in einer Längsrichtung des Positionierungsriemens (41, 41b, 41c, 41d) bewegt, um einen Abstand zwischen einem Verbindungsende des Positionierungsriemens (41, 41b, 41c, 41d), das mit dem Halteelement verbunden ist, und dem Ladegerät-Befestigungssitz (40, 40f) einzustellen.

5. Befestigungsvorrichtung eines drahtlosen Ladegeräts nach Anspruch 3, wobei die Befestigungsschnalle (12) einen weiblichen Klettverschlussgurt und einen männlichen Klettverschlussgurt umfasst, der damit eingreift und eine flauschige Oberfläche aufweist, wobei der weibliche Klettverschlussgurt an einem von dem Befestigungsriemen (42) und dem vorderen Abschnitt des Halteelements angeordnet ist, der männliche Klettverschlussgurt auf dem anderen von dem Befestigungsriemen (42) und dem vorderen Abschnitt des Halteelements angeordnet ist, und der Befestigungsriemen (42) durch den trennbaren Eingriff des weiblichen Klettverschlussgurts und der flauschigen Oberfläche eingestellt und arretiert wird, um die Position des Ladegerät-Befestigungssitzes (40, 40f) einzustellen.

6. Befestigungsvorrichtung eines drahtlosen Ladegeräts nach Anspruch 3, wobei der Positionierungsriemen (41, 41b, 41c, 41d) mit einem mittleren Abschnitt des hinteren Abschnitts des Halteelements über einen Befestigungsriemen (42) verbunden und in Bezug auf das Halteelement drehbar ist.

7. Befestigungsvorrichtung eines drahtlosen Ladegeräts nach Anspruch 3, wobei der Positionierungsriemen (41, 41b, 41c, 41d) lösbar mit dem Schulterhalteabschnitt oder dem hinteren Abschnitt des Halteelements eingreift und die Eingriffsposition eingestellt werden kann.

8. Befestigungsvorrichtung eines drahtlosen Ladegeräts nach Anspruch 2, wobei der vordere Abschnitt des Halteelements eine linke vordere Folie (101) und eine rechte vordere Folie (102) umfasst, und die linke vordere Folie (101) und die rechte vordere Folie (102) beide mit einer Ladeöffnung (11, 11a, 11d, 11e) versehen und trennbar verbunden sind.

9. Befestigungsvorrichtung eines drahtlosen Ladegeräts nach Anspruch 8, wobei die linke vordere Folie (101) und die rechte vordere Folie (102) links und rechts überlappbar und durch Klettverschluss verbunden sind.

10. Befestigungsvorrichtung eines drahtlosen Ladegeräts nach Anspruch 8, wobei eine Außenseite der linken vorderen Folie (101) oder der rechten vorderen Folie (102) mit einem ersten männlichen Klettverschlussband mit einer flauschigen Oberfläche (121) versehen ist, eine Innenseite des anderen von der linken vorderen Folie und der rechten vorderen Folie mit einem weiblichen Klettverschlussband versehen ist, das in die flauschige Oberfläche (121) eingreift, eine Außenseite mit einem zweiten männlichen Klettverschlussband versehen ist, das eine flauschige Oberfläche (122) aufweist, und das andere Ende des Einstellriemens (42) trennbar mit dem zweiten männlichen Klettverschlussband verbunden ist.

11. Befestigungsvorrichtung eines drahtlosen Ladegeräts nach Anspruch 1, wobei das Halteelement einen Schulterhalteabschnitt (30d), eine vordere Folie (10d) und eine hintere Folie (20d) umfasst, die jeweils mit der Vorderseite und der Rückseite des Schulterhalteabschnitts (30d) verbunden sind, das Halteelement als eine symmetrische links-rechts-Struktur konfiguriert ist, der Schulterhalteabschnitt (30d) und die vordere Folie (10d) und die hintere Folie (20d) direkt miteinander verbunden sind und der Schulterhalteabschnitt (30d) und die vordere Folie (10d) und die hintere Folie (20d) eine integrale Struktur sind.

12. Befestigungsvorrichtung eines drahtlosen Ladegeräts nach Anspruch 1, wobei das Halteelement einen Schulterhalteabschnitt (30d), eine vordere Folie (10d) und eine hintere Folie (20d) umfasst, die jeweils mit der Vorderseite und der Rückseite des Schulterhalteabschnitts (30d) verbunden sind, wobei die vordere Folie (10d) von beiden Seiten als eine Zwei-Hälften-Struktur konfiguriert ist, wobei eine Reißverschlussstruktur (50) zwischen den beiden Seiten angeordnet ist, wobei das Anbringen und Abnehmen des Halteelements durch Betätigen der Reißverschlussstruktur erreicht werden kann.

13. Befestigungsvorrichtung eines drahtlosen Ladegeräts nach Anspruch 5, wobei das Halteelement einen Schulterhalteabschnitt (30d), eine vordere Folie (10d) und eine hintere Folie (20d) umfasst, die jeweils mit der Vorderseite und der Rückseite des Schulterhalteabschnitts (30d) verbunden sind, die vordere Folie (10d) einen oberen Abschnitt und einen beabstandeten unteren Abschnitt umfasst, die Ladeöffnung (11d) den oberen Abschnitt der vorderen Folie (10d) von dem unteren Abschnitt der vorderen Folie (10d) trennt und die flauschige Oberfläche auf dem unteren Abschnitt der vorderen Folie (10d) angeordnet ist.

14. Befestigungsvorrichtung eines drahtlosen Ladegeräts nach Anspruch 11 oder 12 oder 13, wobei der Positionierungsriemen (41d) als ein Riemen aus einem dehnbaren Material konfiguriert ist und ein Ende des Positionierungsriemens (41d) über einen Befestigungsdruckknopf (43d) mit der hinteren Folie (20d) verbunden ist, wobei der Befestigungsdruckknopf (43d) in der Mitte der hinteren Folie (20d) angeordnet ist, wobei der Positionierungsriemen (41d) selektiv von der linken Schulter oder der rechten Schulter umgangen werden kann, sodass der Befestigungssitz des Ladegeräts auf die linke oder rechte Seite des Körpers des Patienten eingestellt werden kann.

15. Drahtlose Ladevorrichtung, wobei die drahtlose Ladevorrichtung ein drahtloses Ladegerät und die Befestigungsvorrichtung (100, 200, 300, 400, 500, 600, 700, 800, 900, 1000) des drahtlosen Ladegeräts nach einem der Ansprüche 1 bis 14 umfasst, das drahtlose Ladegerät abnehmbar an dem Ladegerät-Befestigungssitz (40, 40f) angebracht ist und das drahtlose Ladegerät ein Ladefeld erzeugen kann, um die implantierte medizinische Vorrichtung aufzuladen.

## Revendications

1. Dispositif de fixation (100, 200, 300, 400, 500, 600, 700, 800, 900, 1000) d'un chargeur sans fil pour un dispositif médical implanté, le dispositif de fixation comprenant un élément de support pouvant être porté sur le corps d'un patient et une structure de réglage reliée à l'élément de support, un siège de fixation du chargeur (40, 40f) est relié à l'un parmi l'élément de support et la structure de réglage, la structure de réglage comprend une sangle de réglage (41, 41b, 41c, 41d, 42, 62) et une boucle de fixation (12, 61) venant en prise avec la sangle de réglage (41, 41b, 41c, 41d, 42, 62), une extrémité de la sangle de réglage (41, 41b, 41c, 41d, 42, 62) est reliée à l'élément de support, l'autre extrémité de la sangle de réglage (41, 41b, 41c, 41d, 42, 62) amène de manière opérationnelle l'élément de support ou le siège de fixation du chargeur (40, 40f) à être réglé à une position dans laquelle le siège de fixation du chargeur (40, 40f) correspond au dispositif médical implanté, et une position dans laquelle la sangle de réglage vient en prise avec la boucle de fixation peut être réglée et verrouillée, dans lequel le siège de fixation du chargeur (40, 40f) est disposé sur la sangle de réglage (41, 41b, 41c, 41d, 42), une extrémité de la sangle de réglage (41, 41b, 41c, 41d, 42) est reliée à une partie d'épaule ou une partie arrière de l'élément de support, l'autre extrémité de la sangle de réglage (41, 41b, 41c, 41d, 42) amène de manière opérationnelle le siège de fixation du chargeur (40, 40f) à être réglé à une position dans laquelle le siège de fixation du chargeur (40, 40f) correspond au dispositif médical implanté, et l'autre extrémité de la sangle de réglage (41, 41b, 41c, 41d, 42) peut être réglée et verrouillée au niveau de la partie avant de l'élément de support, **caractérisé en ce que**,
l'élément de support comprend une partie de liaison gauche (21, 21a, 21e) et une partie de liaison droite (22, 22a, 22f), les côtés inférieurs d'une partie avant de l'élément de support et une partie arrière de l'élément de support sont reliés par l'intermédiaire de la partie de liaison gauche (21, 21a, 21e) et de la partie de liaison droite (22, 22a, 22f), la partie de liaison gauche (21, 21a, 21e) et la partie de liaison droite (22, 22a, 22f) sont utilisées pour former un manchon d'épaule à supporter sous l'aisselle du patient, une extrémité de la sangle de réglage (41, 41b, 41c, 41d, 42) est reliée à la partie d'épaule de l'élément de support ou à la partie arrière de l'élément de support, et l'autre extrémité de la sangle de réglage (41, 41b, 41c, 41d, 42) est réglée et verrouillée au niveau de la partie avant de l'élément de support.

2. Dispositif de fixation d'un chargeur sans fil selon la revendication 1, dans lequel la partie avant de l'élément de support est pourvue d'une ouverture de charge (11, 11a, 11d, 11e), l'ouverture de charge (11, 11a, 11d, 11e) correspond à une position du dispositif médical implanté, et le siège de fixation du chargeur (40, 40f) peut être réglé dans l'ouverture de charge (11, 11a, 11d, 11e).

3. Dispositif de fixation d'un chargeur sans fil selon la revendication 1, dans lequel la sangle de réglage (41, 41b, 41c, 41d, 42) comprend une sangle de positionnement (41, 41b, 41c, 41d) reliant la partie d'épaule de l'élément de support ou la partie arrière de l'élément de support au siège de fixation du chargeur (40, 40f) et une sangle de fixation (42) reliant le siège de fixation du chargeur (40, 40f) à la partie avant de l'élément de support, et la sangle de fixation (42) peut être réglée et verrouillée au niveau de la partie avant de l'élément de support.

4. Dispositif de fixation d'un chargeur sans fil selon la revendication 3, dans lequel la sangle de positionnement (41, 41b, 41c, 41d) est conçue comme une courroie élastique extensible ou est pourvue d'un dispositif de réglage de longueur, et le dispositif de réglage de longueur se déplace dans une direction longitudinale de la sangle de positionnement (41, 41b, 41c, 41d) pour régler une distance entre une extrémité de liaison de la sangle de positionnement (41, 41b, 41c, 41d) reliée à l'élément de support et le siège de fixation du chargeur (40, 40f).

5. Dispositif de fixation d'un chargeur sans fil selon la revendication 3, dans lequel la boucle de fixation (12) comprend une courroie de système de fermeture autoagrippante femelle et une courroie de système de fermeture autoagrippante mâle venant en prise avec celle-ci et ayant une surface pelucheuse, la courroie de système de fermeture autoagrippante femelle est disposée sur l'une parmi la sangle de fixation (42) et de la partie avant de l'élément de support, la courroie de système de fermeture autoagrippante mâle est disposée sur l'autre sangle de fixation (42) et la partie avant de l'élément de support, et la sangle de fixation (42) est réglée et verrouillée par l'intermédiaire de la mise en prise séparable de la courroie de système de fermeture autoagrippante femelle et de la surface pelucheuse pour régler la position du siège de fixation du chargeur (40, 40f).

6. Dispositif de fixation d'un chargeur sans fil selon la revendication 3, dans lequel la sangle de positionnement (41, 41b, 41c, 41d) est reliée à une partie centrale de la partie arrière de l'élément de support par l'intermédiaire d'un bouton de fixation (42) et peut tourner par rapport à l'élément de support.

7. Dispositif de fixation d'un chargeur sans fil selon la revendication 3, dans lequel la sangle de positionnement (41, 41b, 41c, 41d) est en prise de manière séparable avec la partie de support d'épaule ou la partie arrière de l'élément de support, et la position de mise en prise peut être réglée.

8. Dispositif de fixation d'un chargeur sans fil selon la revendication 2, dans lequel la partie avant de l'élément de support comprend une feuille avant gauche (101) et une feuille avant droite (102), et la feuille avant gauche (101) et la feuille avant droite (102) sont toutes les deux pourvues d'une ouverture de charge (11, 11a, 11d, 11e) et reliées de manière séparable.

9. Dispositif de fixation d'un chargeur sans fil selon la revendication 8, dans lequel la feuille avant gauche (101) et la feuille avant droite (102) peuvent se chevaucher à gauche et à droite et sont reliées de manière autoagrippante.

10. Dispositif de fixation d'un chargeur sans fil selon la revendication 8, dans lequel un côté extérieur de l'une parmi la feuille avant gauche (101) et la feuille avant droite (102) est pourvu d'une première courroie de système de fermeture autoagrippante mâle ayant une surface pelucheuse (121), un côté intérieur de l'autre parmi la feuille avant gauche et la feuille avant droite est pourvu d'une courroie de système de fermeture autoagrippante femelle venant en prise avec la surface pelucheuse (121), un côté extérieur est pourvu d'une seconde courroie de système de fermeture autoagrippante mâle ayant une surface pelucheuse (122), et l'autre extrémité de la sangle de réglage (42) est reliée de manière séparable à la seconde courroie de système de fermeture autoagrippante mâle.

11. Dispositif de fixation d'un chargeur sans fil selon la revendication 1, dans lequel l'élément de support comprend une partie de support d'épaule (30d), une feuille avant (10d) et une feuille arrière (20d) respectivement reliées à l'avant et à l'arrière de la partie de support d'épaule (30d), l'élément de support est conçu comme une structure symétrique gauche-droite, la partie de support d'épaule (30d) et la feuille avant (10d) et la feuille arrière (20d) sont directement reliées ensemble, et la partie de support d'épaule (30d) et la feuille avant (10d) et la feuille arrière (20d) sont une structure solidaire.

12. Dispositif de fixation d'un chargeur sans fil selon la revendication 1, dans lequel l'élément de support comprend une partie de support d'épaule (30d), une feuille avant (10d) et une feuille arrière (20d) respectivement reliées à l'avant et à l'arrière de la partie de support d'épaule (30d), la feuille avant (10d) est conçue comme une structure en deux parties des deux côtés, une structure de fermeture à glissière (50) est disposée entre les deux côtés, la mise en place et le retrait de l'élément de support peuvent être réalisés en actionnant la structure de fermeture à glissière.

13. Dispositif de fixation d'un chargeur sans fil selon la revendication 5, dans lequel l'élément de support comprend une partie de support d'épaule (30d), une feuille avant (10d) et une feuille arrière (20d) respectivement reliées à l'avant et à l'arrière de la partie de support d'épaule (30d), la feuille avant (10d) comprend une partie supérieure et une partie inférieure espacées l'une de l'autre, l'ouverture de charge (11d) sépare la partie supérieure de la feuille avant (10d) de la partie inférieure de la feuille avant (10d), la surface pelucheuse est disposée sur la partie inférieure de la feuille avant (10d).

14. Dispositif de fixation d'un chargeur sans fil selon la revendication 11 ou 12 ou 13, dans lequel la sangle de positionnement (41d) est conçue comme une sangle d'un matériau extensible, et une extrémité de la sangle de positionnement (41d) est reliée à la feuille arrière (20d) par l'intermédiaire d'un bouton de fixation (43d), le bouton de fixation (43d) est disposé au milieu de la feuille arrière (20d), la sangle de positionnement (41d) contourne sélectivement l'épaule gauche ou l'épaule droite de sorte que le siège de fixation du chargeur peut être réglé au côté gauche ou au côté droit du corps du patient.

15. Dispositif de charge sans fil, le dispositif de charge sans fil comprenant un chargeur sans fil et le dispositif de fixation (100, 200, 300, 400, 500, 600, 700, 800, 900, 1000) du chargeur sans fil selon l'une quelconque des revendications 1 à 14, le chargeur sans fil est monté de manière amovible sur le siège de fixation du chargeur (40, 40f), et le chargeur sans fil peut générer un champ de charge pour charger le dispositif médical implanté.
